# EUROPEAN PATENT APPLICATION

(11) **EP 0 974 346 A2**
(43) Date of publication of application: **26.01.2000**
(21) Application number: 99830475.2
(22) Date of filing: 23.07.1999
(51) Int. Cl.: A61K 9/48, A61J 3/07

(54) **Self-sealing capsule and method of producing same**

(30) Priority: 23.07.1998 IT RM980488
(71) Applicant: Nyl Service s.r.l., 00191 Roma (IT)
(72) Inventor: Sacchetti, Antonio, 00189 Roma (IT)
(74) Representative: Sarpi, Maurizio

(57) **Abstract**

In order to seal the capsules which are filled with a medicine where the carrier is a liquid or a colloidal system such a sol or gelatine, some additives are added capable of acting on or interacting with the same carrier of the proximate principle so that an increase in the density of the content of the capsule occurs at least at the contact area with the inside wall of the capsule. Otherwise, if such content of the capsule seeps by capillarity through the junction line between head and body of the capsule, the sealing of such head to such body of the capsule is caused by evaporation of the volatile fraction of the content so that a perfect sealing is obtained.

## Description

The present invention relates to pharmaceutics and more particularly the production of operculi containing mono- or two-phase liquids or colloidal systems such as sol and gelatine, wherein the sealing means is the liquid or the dispersant acting as carrier of the proximate principle.

As known, the term operculum indicates the small capsule in the form of small cylinder with rounded ends consisting of two portions engaging with each other that are filled with a variety of medicines generally in the form of dust or granules, more often liquids, sols or emulsions.
It should be appreciated that, as the two portions forming the operculum are made of rigid gelatine or water dispersible or soluble cellulose capable of dissolving readily in the stomach upon ingestion, the carriers used should be of course limited to those oleaginous or liposoluble in order to prevent the whole shell from being dissolved.
Once the two portions forming the capsule are engaged with each other, the latter has a closure line which runs along the equator in most cases and can be seen on the outside. Such a configuration can be readily disassembled just by applying a light pressure by the fingers on both portions forming the capsule and pulling the same until they are disjoined from each other. It is self-evident that in case the content is a more or less viscous or gelatinous liquid, the latter is quite likely to leak or percolate through the capsule with the result that the proximate principle contained therein is lost and the outside surface become dirty and tacky.
The currently used technology solves such a problem by providing, among other solutions, a band of gelatine along the closure line or merging the head and the body of the capsule with hydroalcoholic mixtures followed by the evaporation of the sealant in a aerated volute. In some cases a sealing lacquer is also used.
Capsules are also produced where the body perfectly mates with the head to limit leakage and facilitate the sealing. Also in this case, however, even considerable loss may occur in spite of a technique which is very expensive, elaborate, generally complicated, and in some cases needing specific equipments.
European Patent Application EP 0 573 978 A1 discloses capsules provided at their outside surface with three layers of foil material acting as sealing means as well as a method of producing such capsules. The limit of such patent is that very expensive machines are needed to make the capsules.

The present invention seeks to overcome the problems of prior art by providing capsules having self-sealing characteristics and capable of keeping the content unchanged even at higher temperatures than the room temperature.
Another object of the invention is to provide a method of producing such self-sealing capsules at low cost and at a production rate so far unexpected.
These objects are accomplished by adding some additives to the content of the capsules or operculi of the above-mentioned type containing liquid or a colloidal system so that the liquid or the dispersant acting as carrier of the proximate principle may perform the function of sealing means.
In other words the inventive step involved by the present invention is to add some additives to the liquid, sol or gelatine contained in the capsule and acting as carrier of the proximate principle in order to increase the density or viscosity, the dropping point or melting point of the capsule content as well as to give the latter thixotropic properties or evaporate to change the organoleptic properties or the physical state of the carrier itself so that said carrier of the proximate principle:
a) grows thick enough to act as a plug for the residual still fluid liquid upon contacting the walls of the capsule; or
b) loses the volatile fraction by evaporation after passing by capillarity the wall of the operculum and coming into contact with air so as to melt head and body of the capsule and to seal it.

Also the residual liquid sets with time and remains stable up to temperatures of 35-40°C.

Further features and advantages of the invention will result from the following detailed description of a preferred not limiting embodiment disclosed only by way of example.
According to the invention the self-sealing property is given to the capsule of the present invention by the following substances: animal waxes and fats, vegetable waxes and fats, triglycerides, fatty alcohols, fatty acids, fatty esters, fatty ethers, gelling silicones, volatile silicones, water-soluble silicones, lacquers, bees-wax, propolis, spermaceti, anionic tensides, non-ionic tensides, amphoteric tensides, cationic tensides, ethyl alcohol, isopropyl alcohol, methylal, microcrystalline cellulose, adsorbents, adsorbents derived from particular, processed starches such as tapioca flour, carbomers, mucilages.
One or more of these substances or a combination of some or all of them may be used.
The substances listed above are suitable as they grow thick after a determined time and do not wet the walls of the capsule because of their determined surface-active characteristics and do not pass therethrough by capillarity which is the cause of their seeping through the capsules.
However, if the seeping of such substances of the present invention such as ethyl alcohol, isopropyl alcohol or methylal by capillarity through the wall of the capsule occurs in any case, the volatile fraction of the liquid or fluid or gel substance coming into contact with air is caused to evaporate and at the same time to melt the head and body portions of the capsule, thus providing their sealing.
The use of the substances including those mentioned above gives sealing properties to the whole medicine contained in the capsule.
More particularly, when the liquid comes into contact with the inside wall of the capsule and seeps by capillarity through its head and body, an increase in the density of the liquid is caused on the inner wall of the capsule, with the result of its possible extension to the whole inside volume of the capsule.
Experiments have proved that, in case the carrier of the proximate principle is a liquid, the whole capsule with hardened liquid keeps its stability which is needed for ensuring the maximum effectiveness of the carried proximate principle up to temperatures ranging between 35°C and 40°C.
The inherent nature of the substances used as "sealing" additives allows the present invention to be applied to the encapsulation of substances including: vitamins, vegetable oils, animal oils, essential oils, mineral oils, perfumes, drugs, and antibiotics.
It should be understood that the present invention may be used in a wide range of applications and the above description relates only to a preferred embodiment thereof. Accordingly, any application of the present invention in a different field without involving an inventive step will not depart from the scope of the present industrial invention.

## Claims

1. A capsule or operculum containing liquids or colloidal systems such as sol or gelatines, characterized in that said liquids or systems are self-sealing as they include additives capable of acting on or interacting with the liquid or the dispersant which is the carrier of the proximate principle so that said carrier of the proximate principle,
a) grows thick enough to act as a plug for the residual still fluid liquid upon contacting the walls of the capsule; or
b) loses the volatile fraction by evaporation after passing by capillarity the wall of the operculum and coming into contact with air so as to melt head and body of the capsule and to seal it.

2. The capsule of claim 1, characterized in that said additives interact with the carrier of the proximate principle and increase its density or viscosity, the dropping point or melting point as well as give said carrier thixotropic properties or evaporate to change the organoleptic properties or the physical state of the carrier itself.

3. The capsule of claims 1 and 2, characterized in that the additive include animal waxes and fats, vegetable fats, triglycerides, fatty alcohols, fatty acids, fatty esters, fatty ethers, gelling silicones, volatile silicones, water-soluble silicones, lacquers, bees-wax, propolis, spermaceti, anionic tensides, non-ionic tensides, amphoteric tensides, cationic tensides, ethyl alcohol, isopropyl alcohol, methylal, microcrystalline cellulose, adsorbents, adsorbents derived from particular, processed starches such as tapioca flour, carbomers, mucilages.

4. The capsule of the preceding claims, characterized in that the additives are present according to *quantum sufficit.*

5. The capsule of the preceding claims, characterized in that the additive are used individually.

6. The self-sealing capsule of the preceding claims, characterized in that the additive are used in combination of two or more of them.

7. The self-sealing capsule of the preceding claims, characterized in that the substances capable of being carried include vitamins, vegetable oils, animal oils, essential oils, mineral oils, perfumes, drugs, and antibiotics.

8. A method of providing self-sealing capsules for medicines, dietary means or cosmetics, characterized in that there is provided the step of adding enough additives to the liquid product or colloidal system which is the carrier of the proximate principle so that the latter interacting with said additives
a) grows thick enough to act as a plug for the residual still fluid liquid upon contacting the walls of the capsule; or
b) loses the volatile fraction by evaporation after passing by capillarity the wall of the operculum and coming into contact with air so as to melt head and body of the capsule and to seal it.

9. The method of the preceding claim, characterized in that the additive is added to the carrier upon filling the capsule.
